# EUROPEAN PATENT APPLICATION

(11) **EP 3 275 865 A1**
(43) Date of publication of application: **31.01.2018**
(21) Application number: 16181910.7
(22) Date of filing: 29.07.2016
(51) Int. Cl.: C07D 213/81, C07C 233/56, C07C 237/30, C07D 495/04, H01L 51/00

(54) **AMIDE-BASED HOLE-TRANSPORTING OR HOLE-INJECTING MATERIALS**

(71) Applicant: Ludwig-Maximilians-Universität München, 80539 München (DE)
(72) Inventor: Petrus, Michiel Leonardus, 80339 München (DE); Docampo, Pablo, 81375 München (DE)
(74) Representative: Vossius & Partner Patentanwälte Rechtsanwälte mbB

(57) **Abstract**

The present invention provides a hole-transporting or hole-injecting material for an optoelectronic device, comprising a compound of the formula (I) and/or a compound of the formula (II) 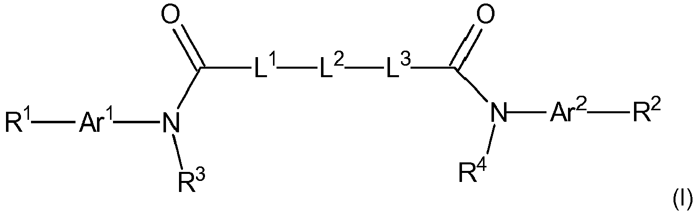 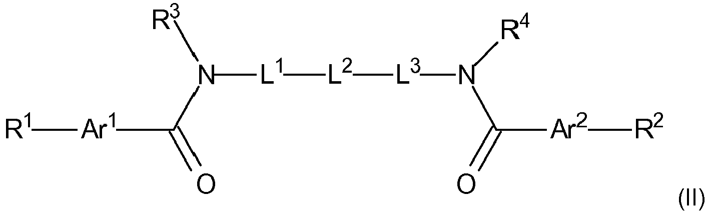 wherein:
Ar¹ and Ar² independently represent an optionally substituted arylene group, an optionally substituted heteroarylene group or an optionally substituted fluorenylene group;
R¹ and R² independently represent hydrogen or a group -NAr⁶Ar⁷, wherein Ar⁶ and Ar⁷ are independently an optionally substituted aryl group, an optionally substituted heteroaryl group, or an optionally substituted fluorenylene group, and wherein a direct covalent bond may be present between Ar⁶ and Ar⁷ such that a fused heterocyclic ring system is formed which contains Ar⁶, Ar⁷ and the nitrogen atom to which they are attached;
L¹ and L³ independently represent a bond, an optionally substituted arylene group, an optionally substituted fluorenylene group, or an optionally substituted heteroarylene group;
L² represents a bond, an optionally substituted arylene group, an optionally substituted heteroarylene group, an optionally substituted fluorenylene group, or a group - Ar³-N(Ar⁴)-Ar⁵-, wherein Ar³ and Ar⁵ represent optionally substituted arylene groups, and Ar⁴ represents an optionally substituted aryl group; and
R³ and R⁴ independently represent hydrogen, an alkyl group or an aryl group.

## Description

The present invention relates to electroactive amide-based materials which show excellent characteristics as hole-transporting or hole-injecting materials in optoelectronic devices, in particular in photovoltaic devices such as perovskite solar cells.

Organic hole-transporting materials have been employed extensively in photovoltaic applications. For instance, they find wide application in organic photovoltaic devices and in hybrid solar cells. Introduced in 1998 to solid-state dye-cells, Spiro-OMeTAD (N²,N²,N^{2'},N^{2'},N⁷,N⁷,N^{7'},N^{7'}-octakis(4-methoxyphenyl)-9,9'-spirobi[9H-fluorene]-2,2',7,7'-tetramine) has been mainly used in solid-state hybrid solar cells. Recently, this material has been employed in combination with a perovskite absorber to achieve power conversion efficiencies exceeding 21% and gained international industrial attention.

However, current state-of-the-art hole-transporting materials used in optoelectronic applications, such as Spiro-OMeTAD, PTAA (Poly[bis(4-phenyl)(2,4,6-trimethylphenyl)amine]) or PCDTBT (Poly[[9-(1-octylnonyl)-9H-carbazole-2,7-diyl]-2,5-thiophenediyl-2,1,3-benzothiadiazole-4,7-diyl-2,5-thiophenediyl]), are very expensive (200-3000 EUR per gram). The high cost is attributed to the multi-step synthesis, involving (transition) metal catalysed cross-coupling reactions, stringent reaction conditions and extensive product purification. These materials are challenging to introduce in commercial applications, as their cost contribution is already higher than the total cost of a commercially available module.

Multiple alternatives have been reported in literature with the objective of replacing in particular Spiro-OMeTAD. These materials follow two trends; either they outperform Spiro-OMeTAD but achieve a marginal reduction in cost (Saliba, M.; Orlandi, S.; Matsui, T.; Aghazada, S.; Cavazzini, M.; Correa-Baena, J.-P.; Gao, P.; Scopelliti, R.; Mosconi, E.; Dahmen, K.-H.; De Angelis, F.; Abate, A.; Hagfeldt, A.; Pozzi, G.; Graetzel, M.; Nazeeruddin, M. K. Nat. Energy 2016, January, 15017) or the costs are significantly reduced, but the material performance is lower than Spiro-OMeTAD (Petrus, M. L.; Bein, T.; Dingemans, T. J.; Docampo, P. J. Mater. Chem. A 2015, 3 (23), 12159).

Hole transporting materials based on an azomethine backbone (WO 2015/142173 A1) allowed achieving a significant cost reduction cost compared to state-of-the-art materials. Nevertheless, a need remains for materials which can be used as hole-transporting or hole-injecting materials in optoelectronic devices with a high efficiency.

Thus, the present invention provides a compound of the formula (I) and/or a compound of the formula (II) 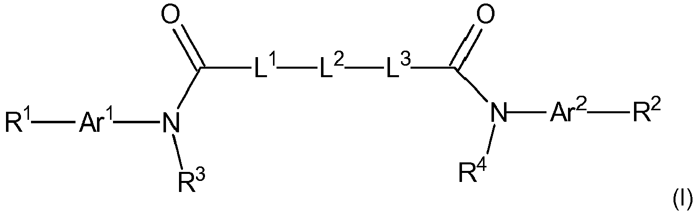 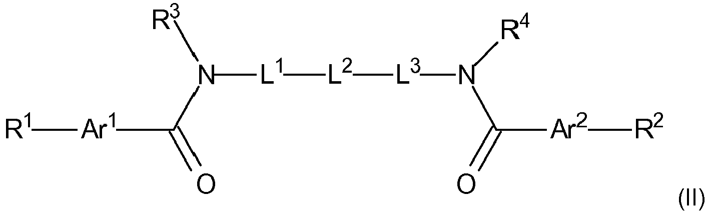 for use as a hole-transporting or hole-injecting material in an optoelectronic device, wherein:
Ar¹ and Ar² independently represent an optionally substituted arylene group, an optionally substituted heteroarylene group or an optionally substituted fluorenylene group;
R¹ and R² independently represent hydrogen or a group -NAr⁶Ar⁷, wherein Ar⁶ and Ar⁷ are independently an optionally substituted aryl group, an optionally substituted heteroaryl group, or an optionally substituted fluorenyl group, and wherein a direct covalent bond may be present between Ar⁶ and Ar⁷ such that a fused heterocyclic ring system is formed which contains Ar⁶, Ar⁷ and the nitrogen atom to which they are attached;
L¹ and L³ independently represent a bond, an optionally substituted arylene group, an optionally substituted heteroarylene group, or an optionally substituted fluorenylene group;
L² represents a bond, an optionally substituted arylene group, an optionally substituted heteroarylene group, an optionally substituted fluorenylene group, or a group -Ar³-N(Ar⁴)-Ar⁵-, wherein Ar³ and Ar⁵ independently represent optionally substituted arylene group, and Ar⁴ represents an optionally substituted aryl group;
R³ and R⁴ independently represent hydrogen, an alkyl group or an aryl group;
and wherein any optionally substituted aryl group, arylene group, heteroaryl group, heteroarylene group, fluorenyl group and fluorenylene group may be substituted by one or more substituents, independently selected from -CN, alkyl, alkoxy, aryl, heteroaryl, halogen, and amino, and wherein two optional adjacent substituents selected from alkyl, alkoxy and amino may be taken together to form a ring fused with the aryl group, arylene group, heteroaryl group, heteroarylene group, fluorenyl group and fluorenylene group to which they are attached.

Moreover, the invention provides an optoelectronic device, such as an organic light emitting diode (OLED) and a photovoltaic device, and in particular a perovskite solar cell, comprising one or more compounds of formula (I) and/or (II) as defined above.

The suitability of compounds containing amide linkers in their backbone as hole-transporting or hole-injecting materials was not obvious since such linkers are only conjugated in the resonance structure and would therefore be expected by a person skilled in the art to lead to a poor charge carrier mobility. However, it was unexpectedly found that the amide group containing compounds of formula (I) or (II) possess very good hole-transporting properties, making them suitable for use in optoelectronic devices. The materials used in the context of the invention can be synthesized in simple and low-cost condensation reactions. These chemistries do not require expensive transition metal catalysts, and can be performed under near ambient conditions, with non-toxic byproducts such as water or HCl. The high synthetic accessibility of these materials reduces the toxic chemical waste and therefore significantly reduces its environmental impact as the synthesis does not require halogenated solvents. Materials based on amides are extremely relevant in industry due to their high durability and strength. For instance, well known polyamides like Nylon or Kevlar are currently produced very cheaply (< 0.5 Eur/g) at the commodity scale. These materials are produced in low-cost condensation reactions, in which water is the only side-product. The materials used in the context of the invention can be purified in a single washing step, making them compatible with easily up-scalable industrial processes. Thus, it is to be expected that the costs of these materials can remain below 10 euros per gram, which results in a negligible contribution in the module cost of optoelectronic devices. Additionally, the compounds of formula (I) and/or (II) above allow the production of optoelectronic devices which outperform those based on a state-of-the-art material as well as the previously developed azomethine-based materials on a like to like comparison.

As noted above, the compound used as a hole-transporting material or a hole-injecting material in an optoelectronic device in accordance with the present invention is a compound of the formula (I) and/or a compound of the formula (II) as shown below.

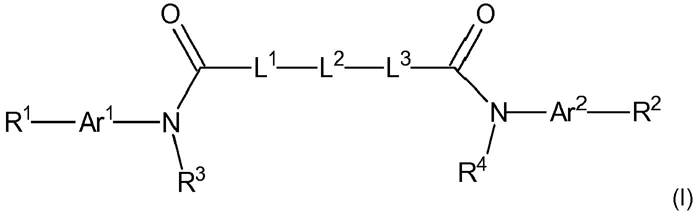

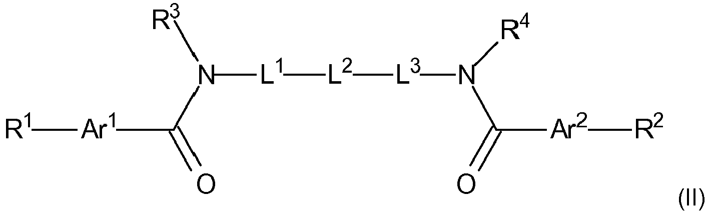

As will be readily understood by the skilled person, the expression "and/or" indicates in this context that (i) one or more compounds of formula (I) can be used, (ii) one or more compounds of formula (II) can be used, or (iii) one or more compounds of formula (I) can be used in combination with one or more compounds of formula (II). Preferably, the compound used as a hole-transporting or hole-injecting material in accordance with the invention is a compound of formula (I).

In formula (I) and (II), and in the preferred variants thereof discussed herein, an aryl group may be a monocyclic aromatic hydrocarbon group or a polycyclic aromatic hydrocarbon group containing two or more rings in a fused ring system. Preferred as aryl groups are those with 6 to 14 ring members. It will be understood that the indicated number of ring members does not include the ring members contained in an optional substituent with a ring structure, or in two optional substituents forming such a ring structure together. More preferred are phenyl and naphtyl, and particularly preferred is phenyl.

An arylene group may be a divalent monocyclic aromatic hydrocarbon group or divalent polycyclic aromatic hydrocarbon group containing two or more rings in a fused ring system, such that the arylene group has two points of attachment to the remainder of the molecule. Preferred as arylene groups are those with 6 to 14 ring members. It will be understood that the indicated number of ring members does not include the ring members contained in an optional substituent with a ring structure, or in two optional substituents forming such a ring structure together. More preferred are phenylene and naphtylene, and particularly preferred is phenylene.

A heteroaryl group may be a monocyclic aromatic group or a polycyclic aromatic group containing two or more rings in a fused ring system. Preferred as heteroaryl groups are those with 5 to 14 ring members in a monocyclic aromatic group or a polycyclic aromatic group, and more preferred as heteroaryl groups are those with 5 to 9 ring members in a monocyclic aromatic group or a polycyclic aromatic group, and even more preferred are those with 5 ring members in a monocyclic aromatic group. It will be understood that the indicated number of ring members does not include the ring members contained in an optional substituent with a ring structure or in two optional substituents forming such a ring structure together. The heteroaryl group comprises one or more, preferably one, two or three, and more preferably one, heteroatom as a ring member in a monocyclic aromatic group or a polycyclic aromatic group. Preferably, the heteroatoms are independently selected from N, O, S and Se, more preferably from O, S and Se and most preferred as heteroatom is S. Preferred examples of the heteroaryl group are a benzothiadiazolyl group, a furanyl group, a thienyl group, and a selenyl group. More preferred is a thienyl group.

A heteroarylene group may be a divalent monocyclic aromatic group or a divalent polycyclic aromatic group containing two or more rings in a fused ring system, such that the heteroarylene group has two points of attachment to the remainder of the molecule. Preferred as heteroarylene groups are those with 5 to 14 ring members in a monocyclic aromatic group or a polycyclic aromatic group, and more preferred as heteroaryl groups are those with 5 to 9 ring members in a monocyclic aromatic group or a polycyclic aromatic group, and even more preferred are those with 5 ring members in a monocyclic aromatic group. It will be understood that the indicated number of ring members does not include the ring members contained in an optional substituent with a ring structure or in two optional substituents forming such a ring structure together. The heteroarylene group comprises one or more, preferably one, two or three, and more preferably one, heteroatom as a ring member in a monocyclic aromatic group or a polycyclic aromatic group. Preferably, the heteroatoms are independently selected from N, O, S and Se, more preferably from O, S and Se and most preferred as heteroatom is S. Preferred examples of the heteroarylene group are a benzothiadiazolylene group, a furanylene group, a thienylene group, and a selenylene group. More preferred is a thienylene group.

As noted above, any optionally substituted aryl group, arylene group, heteroaryl group, heteroarylene group, fluorenyl group and fluorenylene group may be substituted by one or more substituents independently selected from -CN, alkyl, alkoxy, aryl, heteroaryl, halogen, and amino. Moreover, two adjacent substituents selected from alkyl, alkoxy and amino may be taken together to form a ring fused with the aryl group, arylene group, heteroaryl group, heteroarylene group, fluorenyl group and fluorenylene group to which they are attached.

For the alkyl group as a substituent, preferred are C1-C6 alkyl groups, more preferred is a methyl group. For the alkoxy group as a substituent, preferred is a C1-C6 alkoxy group, more preferred is a methoxy group. For the aryl group and the heteroaryl group, the preferred number of ring members and the preferred number and nature of heteroatoms has been discussed above. Also in this context, preferred as aryl groups are phenyl and naphtyl, and particularly preferred is phenyl, and preferred examples of the heteroaryl group are a benzothiadiazolyl group, a furanyl group, a thienyl group, and a selenyl group, and particularly preferred is a thienyl group. For the halogen atom as a substituent, preferred is F, Cl and Br, and particularly preferred is Cl. In cases where two adjacent substituents selected from alkyl, alkoxy and amino are taken together to form a fused ring, it is preferred that two alkoxy groups are taken together to form a divalent substituent of the formula -O-(CH₂)ₙ-O-, wherein n is 1, 2 or 3, preferably 2.

Preferred optional substituents are alkoxy, such as more preferably methoxy, alkyl, such as more preferably methyl, halogen, and amino, and particularly preferred is alkoxy, most preferred methoxy.

Unless indicated otherwise in a specific context, any optionally substituted aryl group, arylene group, heteroaryl group, heteroarylene group, fluorenyl group and fluorenylene group is preferably unsubstituted or carries 1 or 2 substituents.

In formula (I) and (II), and in the preferred variants thereof discussed herein, Ar¹ and Ar² independently represent an optionally substituted arylene group, an optionally substituted heteroarylene group, or an optionally substituted fluorenylene group. Preferred options for the optionally substituted arylene group and the optionally substituted heteroarylene group are as discussed above. In this context, it will be understood that the term "optionally substituted" used in the definition of Ar¹ and Ar², respectively, does not relate to the groups R¹ and R², respectively, which are separately defined herein. Thus, the optional substituent of Ar¹ or Ar², if present, would be attached to Ar¹ and Ar² at a position other than that of R¹ or R², respectively. Among the options available for Ar¹ and Ar², the optionally substituted arylene group and the optionally substituted heteroarylene group are preferred, and more preferred is the optionally substituted arylene group, in particular the optionally substituted phenylene, and even further preferred is an unsubstituted arylene group, in particular phenylene.

In formula (I) and (II), and in the preferred variants thereof discussed herein, R¹ and R² independently represent hydrogen or a group -NAr⁶Ar⁷, wherein Ar⁶ and Ar⁷ are independently an optionally substituted aryl group, an optionally substituted heteroaryl group, or an optionally substituted fluorenyl group. Preferred options for the optionally substituted aryl group and the optionally substituted heteroaryl group are as discussed above. Between Ar⁶ and Ar⁷, a direct covalent bond may be present such that a fused heterocyclic ring system is formed which contains Ar⁶, Ar⁷ and the nitrogen atom to which they are attached. An example of such a heterocyclic ring system is a carbazolyl group. It will be understood that the optional substituents which may be attached to Ar⁶ and Ar⁷ can also be present on the heterocyclic ring system formed by covalently bonding Ar⁶ and Ar⁷. Among the options available for R¹ and R², the group -NAr⁶Ar⁷ is preferred.

When R¹ or R² or both represent a group -NAr⁶Ar⁷, it is preferred that Ar⁶ and Ar⁷ are independently an optionally substituted aryl group, and more preferred that they are an optionally substituted phenyl group which may carry 1 or 2, still more preferably 1 substituent. Preferred options for the substituent are as defined above, particularly preferred is methoxy. It is also preferred that the optional covalent bond between Ar⁶ and Ar⁷ is absent. Most preferably, Ar⁶ and Ar⁷ are p-methoxyphenyl.

In formula (I) and (II), and in the preferred variants thereof discussed herein, L¹ and L³ independently represent a bond, an optionally substituted arylene group, an optionally substituted heteroarylene group, or an optionally substituted fluorenylene group. Preferred options for the optionally substituted arylene group and the optionally substituted heteroarylene group are as discussed above. If L¹, L³, or both, represent an optionally substituted arylene group or an optionally substituted heteroarylene group, it is preferred that a substituent be absent.

Among the options available for L¹ and L³, it is preferred that L¹ and L³ independently represent a bond or an optionally substituted heteroarylene group, and more preferred that both L¹ and L³ represent a bond. In this later case, the moiety -L¹-L²-L³- in formula (I) and (II) is reduced to -L²-.

In formula (I) and (II), and in the preferred variants thereof discussed herein, L² represents a bond, an optionally substituted arylene group, an optionally substituted heteroarylene group, an optionally substituted fluorenylene group, or a group -Ar³-N(Ar⁴)-Ar⁵-, wherein Ar³ and Ar⁵ independently represent an optionally substituted arylene group, and Ar⁴ represents an optionally substituted aryl group. Preferred options for the optionally substituted aryl group, the optionally arylene group and the optionally substituted heteroarylene group are as discussed above. Among the options available for L², it is preferred that L² is an optionally substituted heteroarylene group.

It is more preferred that L² represents a moiety of the formula (III): 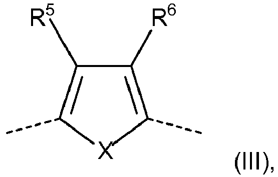 wherein
X is a heteroatom selected from O, Se and S, and is particularly preferably S,
R⁵ and R⁶ are independently selected from H, -CN, alkyl, alkoxy, aryl, heteroaryl, halogen and amino, and two substituents selected from alkyl, alkoxy and amino may be taken together to form a ring structure fused with the heteroarylene group to which they are attached, and the dashed lines indicate the position where covalent bonds are formed by the divalent moiety L² to its neighboring atoms.

For the alkyl group as R⁵ or R⁶, preferred are C1-C6 alkyl groups, more preferred is a methyl group. For the alkoxy group as R⁵ or R⁶, preferred is a C1-C6 alkoxy group, more preferred is a methoxy group. For the aryl group and the heteroaryl group, the preferred number of ring members and the preferred number and nature of heteroatoms are discussed above. Also in this context, preferred as aryl groups are phenyl and naphtyl, and particularly preferred is phenyl, and preferred examples of the heteroaryl group are a benzothiadiazolyl group, a furanyl group, a thienyl group, and a selenyl group, and particularly preferred is a thienyl group. For the halogen atom as R⁵ or R⁶, preferred is F, Cl and Br, and particularly preferred is Cl. In cases where two substituents selected from alkyl, alkoxy and amino are taken together to form a fused ring, it is preferred that two alkoxy groups are taken together to form a divalent substituent of the formula -O-(CH₂)ₙ-O-, wherein n is 1, 2 or 3, preferably 2.

Preferred options for R⁵ or R⁶ are alkoxy, such as more preferably methoxy, alkyl, such as more preferably methyl, halogen, amino, and the option that two substituents selected from alkyl, alkoxy and amino are taken together to form a fused ring. It is particularly preferred that R⁵ and R⁶ are two alkoxy groups taken together to form a divalent substituent of the formula -O-(CH₂)ₙ-O-, wherein n is 1, 2 or 3, preferably 2.

Thus, in a more preferred embodiment, L² represents a moiety of the formula (IV): 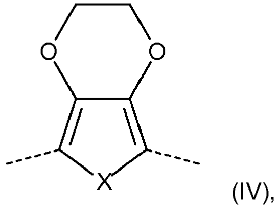 wherein X is a heteroatom selected from O, Se and S, and is particularly preferably S, and the dashed lines indicate the position where covalent bonds are formed by the divalent moiety L² to its neighboring atoms.

In formula (I) and (II), and in the preferred variants thereof discussed herein, R³ and R⁴ independently represent hydrogen, an alkyl group or an aryl group. Preferred as an alkyl group is a C1-C6 alkyl group, and further preferred is methyl. Preferred as an aryl group is phenyl. Among the options available for R³ and R⁴, it is preferred that R³ and R⁴ are hydrogen.

As will be apparent from the above discussion, preferred compounds for use in the context of the present invention are those of the following formulae (Ia) and/or (IIa), more preferably of the formula (Ia) 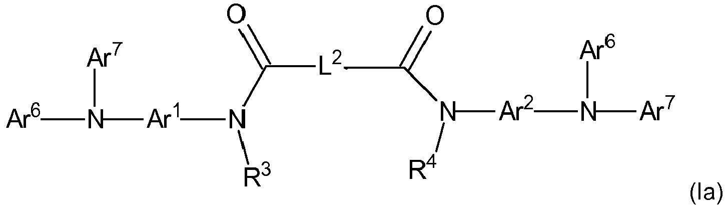 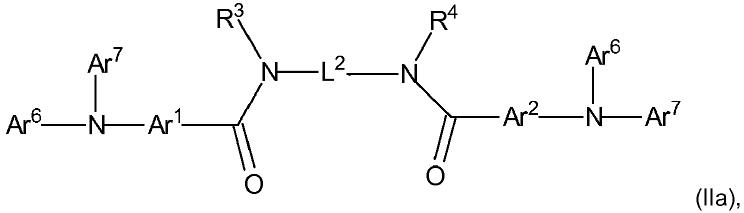 wherein:
L², R³, R⁴, Ar¹, Ar², Ar⁶ and Ar⁷ are defined as above, including preferred embodiments.

In a more preferred embodiment, the following definitions for the variables in formula (Ia) and (IIa) apply:
L² is a moiety of the formula (III), more preferably of the formula (IV) defined above;
R³ and R⁴ are hydrogen;
Ar¹ and Ar² are independently an optionally substituted arylene group, more preferably an optionally substituted phenylene group, and even more preferably a phenylene group;
Ar⁶ and Ar⁷ are, independently from each other, and independently for each occurrence, an optionally substituted aryl group, more preferably an optionally substituted phenyl group; wherein the optionally substituted aryl group and the optionally substituted phenyl group may carry 1 or 2, preferably 1 alkoxy substituent, and wherein more preferably the optionally substituted aryl group and the optionally substituted phenyl group may carry 1 or 2, preferably 1 methoxy substituent.

Preferred exemplary compounds of formula (I) or (II) are illustrated in the following: 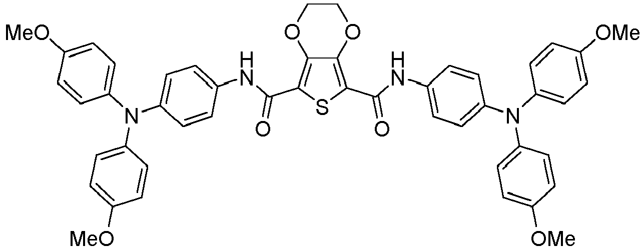 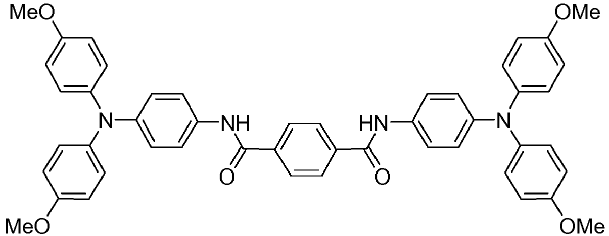 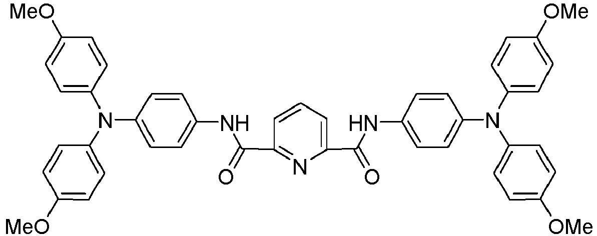 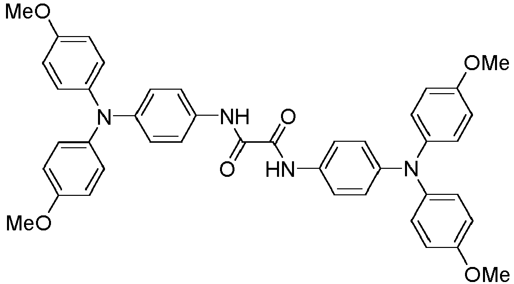 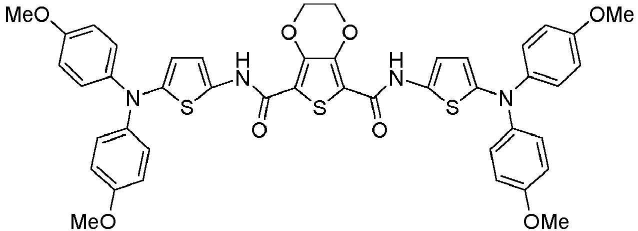 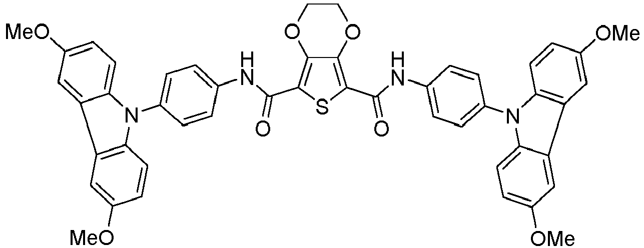 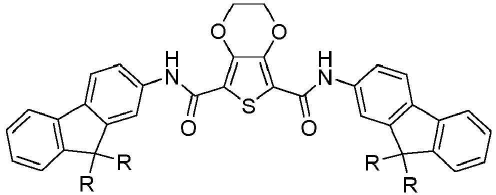 (in this formula, the groups R independently represent H or an optional substituent of a fluorenyl group as defined above, including prefered embodiments); 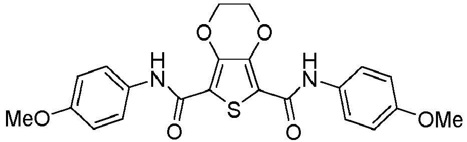 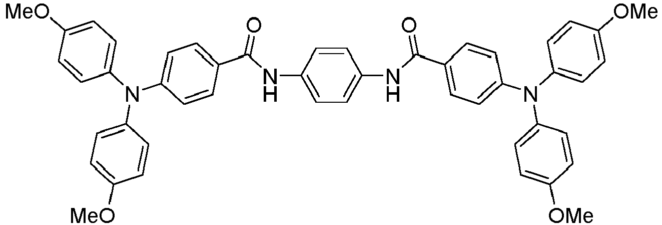 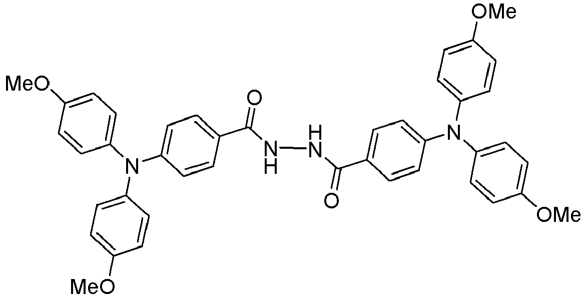 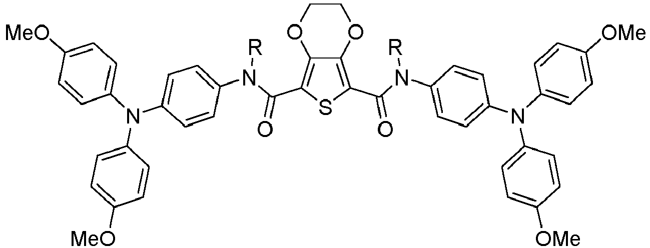 (in this formula, the groups R are independently defined as the groups R³ and R⁴ above, including preferred embodiments);

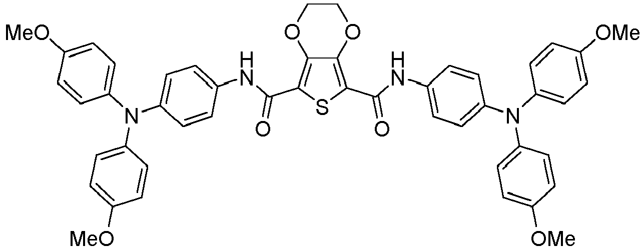

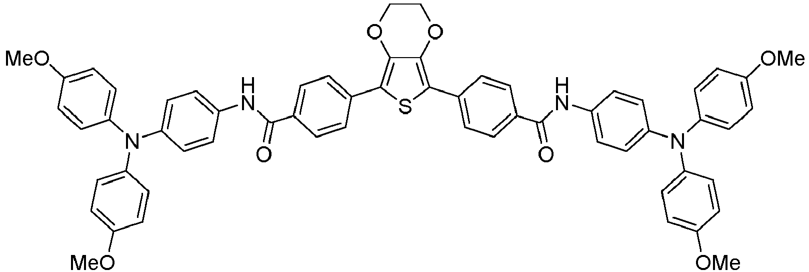

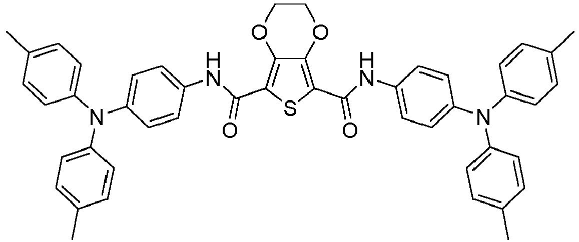

The compounds of formula (I) and of formula (II) are readily accessible via condensation reactions between suitable dicarboxylic acids and amines, or between suitable diamines and carboxylic acids. In accordance with standard methods of organic chemistry, the carboxylic acid groups may be provided in an activated form to facilitate the reaction. Starting materials are commercially available as such, or may be obtained via derivatization reactions from commercially available components. Advantageously, such reactions can be efficiently carried out at high yields, and the high synthetic accessibility of these molecules also reduces the toxic chemical waste and therefore greatly reduces its environmental impact. For example, the synthesis does not have to rely on halogenated solvents or (transition) metal catalysts.

The compounds of formula (I) and of formula (II) have charge-transporting capabilities and may therefore be used as hole transporting or hole injecting material alone. However, it is also possible to use them in combination with additives and/or dopants, e.g. if it is desired to enhance the charge transporting capabilities for an intended application in an optoelectronic device.

For example, in order to apply these above compounds of formula (I) and/or (II) as hole-transporting or hole-injecting materials, a conductivity improving additive is preferably added to the material. The additive is typically of p-type. A typical amount of the additive is 0.1 to 400 mole%, relative to the molar amount of compounds of formula (I) and of formula (II), preferably 10-300 mole%, more preferably 50-200 mole%, such as 100-150 mole%. The additive may be considered as a dopant to the hole-transporting or hole-injecting material; it is noted that the concentration of the additives may typically be much higher than e.g. dopants in semiconductor devices. Such conductivity improving additives are known to the skilled person.

One suitable type of conductivity improving additive is an additive capable of oxidizing the compound of formula (I) and/or (II), and/or to promote an oxidative reaction thereof; in this respect the additive may be considered as an oxidant. In principle any such oxidant can be applied in the present invention singly or in combination, if desired.

As examples of suitable conductivity improving additives, which may be used singly or in combination of two or more, the following may be mentioned: bis(trifluoromethylsulfonyl)imide (TFSI) and its salts, preferably alkali salts, alkaline earth metal salts, other monovalent salts, hydrogen salts or acids, and *N,N*-dialkyl pyrrolidinium salts, such as *N,N*-dimethyl- pyrrolidinium TFSI, *N*-methyl-N-propylpyrrolidinium TSFI, LiTFSI, AgTFSI, or HTFSI; *N,N*-dialkyl pyrrolidinium salts with other anions, such as N,N-dimethyl-pyrrolidinium iodide; N(PhBr)₃SbCl₆; 2,3,5,6-Tetrafluoro-7,7,8,8-tetracyanoquinodimethane (F4TCNQ); and cobalt dopants, such as tris(2-(1H-pyrazol-1-yl)pyridine)cobalt(II) di[hexafluorophosphate] (FK102) or bis(2,6-di(1H-pyrazol-1-yl)pyridine)cobalt(III) tri[bis(trifluoromethane)sulfonimide] (FK269).

Other exemplary additives that may be used in combination with the compounds of formula (I) and/or (II) especially in perovskite solar cells as optoelectronic device are additives for passivating the perovskite surface. Generally these additives are combined with the other optional additives, e.g. the conductivity improving additives. They are typically used in a concentration of 10-1000 mole%, such as 100 mole%, relative to the molar amount of compounds of formula (I) and of formula (II). As examples of such additives, 4-tert-butylpyridine (TBP), pyridine, iodopentafluorobenzene (IPFB), 2,6-lutidine, and 4-methylpyridine may be mentioned.

As explained above, the compounds of formula (I) and of formula (II), as well as their preferred embodiments, can be effectively used as hole-transporting material or hole injecting-material, preferably as a hole-transporting material, in an optoelectronic device. Thus, the present invention also encompasses an optoelectronic device comprising one or more compounds of formula (I) and/or (II), including its preferred embodiments such as compounds of formula (Ia) and/or (IIa). As will be understood by the skilled reader, the one or more compounds of formula (I) and/or (II) will typically contained as hole-transporting material or hole-injecting material, preferably as a hole-transporting material, i.e. they will be present in a position where they can provide this function.

Optoelectronic devices are known as electronic devices which emit light, detect light, or convert light, in particular sunlight, into electric energy. A preferred type of an optoelectronic device in which the compounds of formula (I) and/or (II) can be favorably used as hole-transporting material or hole-injecting material are organic light emitting diodes (OLEDS). Further preferred as photovoltaic devices are solar cells, and particularly preferred are perovskite solar cells, wherein the compounds of formula (I) and/or (II) are typically used as hole-transporting material.

Optoelectronic devices, including those using an organic hole-transporting material or hole-injecting material, and their preparation, are established in the art, and the compounds (I) and/or (II) can be used as an economic and efficient alternative to replace a known hole-transporting material or hole-injecting material, such as Spiro-OMeTAD, in such devices. Reference can be made, e.g., to literature such as "Organic Electronics: Materials, Manufacturing, and Applications", H. Klauk ed., Wiley-VCH, 2006; D. J. Gaspar et al., "OLED Fundamentals: Materials, Devices, and Processing of Organic Light-Emitting Diodes", CRC Press 2015; T. Swetha et al., "Perovskite solar cells based on small molecule hole transporting materials"; J. Mater. Chem. A, 2015,3, 18329-18344, DOI: 10.1039/C5TA02507A.

Generally, the hole-transporting material or hole-injecting material of an optoelectronic device is positioned between an anode and a cathode in, typically in the form of an organic layer. The organic layer may further comprise additives and/or dopants to enhance the conductivity or to passivate a surface, e.g. as described previously. For example, in the case of an OLED a layer functioning as a hole-transporting layer is typically present between the anode, where the holes are generated, and the light emitting layer, where the holes combine with the electrons generated in the cathode to emit light. A hole-injecting material, if present, may be provided e.g. in the form of a hole-injecting layer which is present between the anode and the hole-transporting layer, or a combined hole-injecting/hole-transporting layer may be formed between the anode and the light emitting layer. In the case of a solar cell, such as a perovskite solar cell, a hole-transporting layer is typically present between a layer wherein charges are generated due to irradiation with sunlight (also referred to as active layer), and the electrode where the holes are collected.

In order to incorporate compounds of formula (I) and/or compounds of formula (II) into an optoelectronic device, the compounds can, for example, be deposited using thermal evaporation, or deposited using solution processing where the compounds are dissolved or dispersed, together with other optional components, if present, in a suitable solvent. The solution or suspension can then be applied to an underlying material via conventional techniques such as spin coating, doctor blading, roll coating or the like, followed by the removal of the solvent e.g. via heat, reduced pressure, use of a desiccant, or the like.

The present invention shows that a new type of hole-transporting or hole-injecting materials based on an amide backbone can be employed in optoelectronic devices, e.g. perovskite solar cells. Compared to state-of-the-art materials, these new hole-transporters lead to devices which exceed the performance of previous record-holding materials. In addition, the new materials can be synthesized from readily available starting materials in a low-cost condensation reaction with water or HCl as the only byproducts, greatly simplifying their purification protocol. This leads to a significant reduction in the final price of the product. This is the first demonstration of a hole-transporting material which outperforms the state-of-the-art, while simultaneously being 10 times cheaper to produce.

In the following, a summary is provided of relevant aspects of the invention. As will be understood, those items which contain a back-reference to a preceding item define a preferred embodiment of this preceding item. As will also be understood, these items form a part of the overall disclosure of the invention, and the information provided above equally applies for the subject matter summarized in these items.
1. A compound of the formula (I) or of the formula (II) 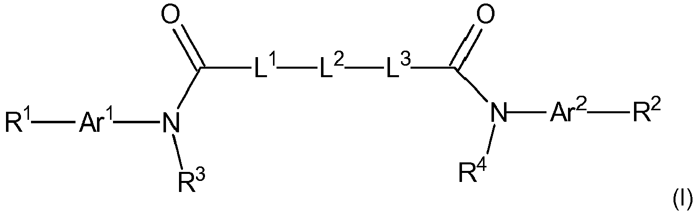 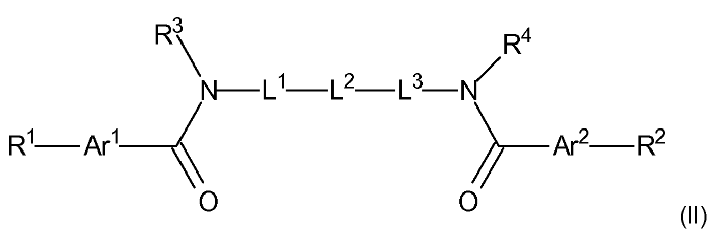 as a hole-transporting or hole-injecting material in an optoelectronic device, wherein:
   Ar¹ and Ar² independently represent an optionally substituted arylene group, an optionally substituted heteroarylene group or an optionally substituted fluorenylene group;
   R¹ and R² independently represent hydrogen or a group -NAr⁶Ar⁷, wherein Ar⁶ and Ar⁷ are independently an optionally substituted aryl group, an optionally substituted heteroaryl group, or an optionally substituted fluorenyl group, and wherein a direct covalent bond may be present between Ar⁶ and Ar⁷ such that a fused heterocyclic ring system is formed which contains Ar⁶, Ar⁷ and the nitrogen atom to which they are attached;
   L¹ and L³ independently represent a bond, an optionally substituted arylene group, an optionally substituted heteroarylene group, or an optionally substituted fluorenylene group;
   L² represents a bond, an optionally substituted arylene group, an optionally substituted heteroarylene group, an optionally substituted fluorenylene group, or a group -Ar³-N(Ar⁴)-Ar⁵-, wherein Ar³ and Ar⁵ independently represent an optionally substituted arylene group, and Ar⁴ represents an optionally substituted aryl group;
   R³ and R⁴ independently represent hydrogen, an alkyl group or an aryl group;
   and wherein any optionally substituted aryl group, arylene group, heteroaryl group, heteroarylene group, fluorenyl group and fluorenylene group may be substituted by one or more substituents, independently selected from -CN, alkyl, alkoxy, aryl, heteroaryl, halogen, and amino, and wherein two adjacent optional substituents selected from alkyl, alkoxy and amino may be taken together to form a ring fused with the aryl group, arylene group, heteroaryl group, heteroarylene group, fluorenyl group and fluorenylene group to which they are attached.
2. The compound in accordance with item 1, wherein
   L¹ and L³ represent a bond;
   L² represents an optionally substituted arylene group, an optionally substituted heteroarylene group, or a group -Ar³-N(Ar⁴)-Ar⁵-, wherein Ar³ and Ar⁵ independently represent an optionally substituted arylene group, and Ar⁴ represents an optionally substituted aryl group.
3. The compound in accordance with item 1 or 2, wherein
   L¹ and L³ represent a bond;
   L² represents an optionally substituted arylene group or an optionally substituted heteroarylene group.
4. The compound in accordance with any of items 1 to 3, wherein
   L¹ and L³ represent a bond;
   L² represents an optionally substituted heteroarylene group.
5. The compound in accordance with any of items 2 to 4, wherein the compound of formula (I) is a compound of formula (Ia) and the compound of formula (II) is a compound of formula (IIa): 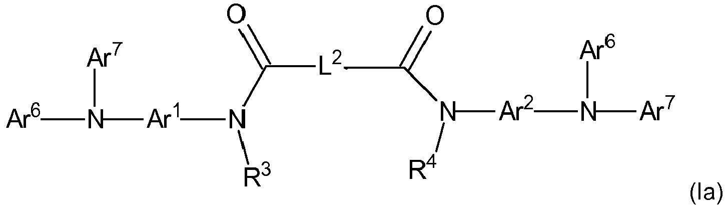 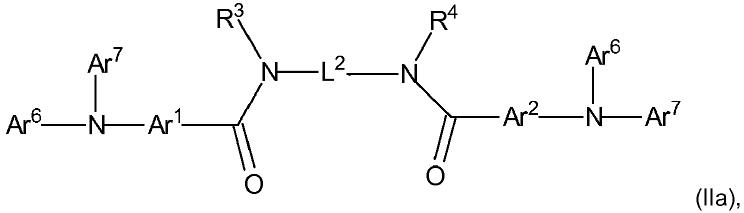 wherein:
   L² is defined as in items 2 to 4, and R³, R⁴, Ar¹, Ar², Ar⁶ and Ar⁷ are defined as in item 1.
6. The compound in accordance with any of items 1 to 5, wherein Ar¹ and Ar² independently represent an optionally substituted arylene or an optionally substituted heteroarylene group.
7. The compound in accordance with item 6, wherein Ar¹ and Ar² independently represent an optionally substituted arylene group.
8. The compound in accordance with any of items 1 to 6 wherein Ar¹ and Ar² independently represent an optionally substituted phenylene group.
9. The compound in accordance with any of items 1 to 8, wherein R¹ and R² independently represent a group -NAr⁶Ar⁷, wherein Ar⁶ and Ar⁷ are independently an optionally substituted aryl group, and wherein a direct covalent bond may be present between Ar⁶ and Ar⁷ such that a fused heterocyclic ring system is formed which contains Ar⁶, Ar⁷ and the nitrogen atom to which they are attached.
10. The compound in accordance with any of items 1 to 9, wherein R¹ and R² independently represent a group -NAr⁶Ar⁷, wherein Ar⁶ and Ar⁷ are independently an optionally substituted phenyl group, and wherein a direct covalent bond may be present between Ar⁶ and Ar⁷ such that a fused heterocyclic ring system is formed which contains Ar⁶, Ar⁷ and the nitrogen atom to which they are attached.
11. The compound in accordance with item 10, wherein R¹ and R² independently represent a group -NAr⁶Ar⁷, wherein Ar⁶ and Ar⁷ are independently an optionally substituted phenyl group.
12. The compound in accordance with any of items 1 to 11, wherein R³ and R⁴ are hydrogen.
13. The compound in accordance with any of items 1 to 12, wherein L² represents a moiety of the formula (III): 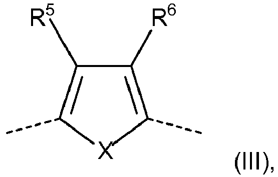 wherein
   X is a heteroatom selected from O, Se and S, and
   R⁵ and R⁶ are independently selected from H, -CN, alkyl, alkoxy, aryl, heteroaryl, halogen and amino, and two substituents selected from alkyl, alkoxy and amino may be taken together to form a ring structure fused with the heteroarylene group to which they are attached, and the dashed lines indicate the position where covalent bonds are formed by the divalent moiety L² to its neighboring atoms.
14. The compound in accordance with item 13, wherein L² represents a moiety of the formula (IV): 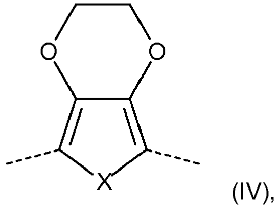 wherein
   X is a heteroatom selected from O, Se and S, and the dashed lines indicate the position where covalent bonds are formed by the divalent moiety L² to its neighboring atoms.
15. The compound in accordance with item 13 or 14, wherein X is S.
16. The compound in accordance with any of items 1 to 15 for use as a hole-transporting or hole-injecting material in an optoelectronic device.
17. A hole-transporting or hole-injecting material for an optoelectronic device comprising one or more compounds in accordance with any of items 1 to 15.
18. Use of one or more compounds of formula (I), (Ia), (II) and/or (IIa) in accordance with any of items 1 to 15 as a hole-transporting or hole-injecting material in an optoelectronic device.
19. Use in accordance with item 18, wherein the hole-transporting or hole-injecting material comprises a mixture of two or more compounds of formula (I) or (Ia), a mixture of two or more compounds of formula (II) or (IIa), or a mixture of one or more compounds of formula (I) or (Ia) with one or more compounds of formula (II) or (IIa).
20. Use in accordance with item 18 or 19, wherein the compound of formula (I), (Ia), (II) and/or (IIa) is contained in an organic film in the optoelectronic device.
21. Use in accordance with any of items 18 to 20, wherein the optoelectronic device is selected from an organic light emitting diode (OLED) and a photovoltaic device.
22. Use in accordance with item 21, wherein the optoelectronic device is a solar cell.
23. Use compound in accordance with item 22, wherein the solar cell is a perovskite solar cell.
24. An optoelectronic device, comprising one or more compounds of formula (I), (Ia), (II) and/or (IIa) in accordance with any of items 1 to 15.
25. Optoelectronic device in accordance with item 24, wherein the compound of formula (I), (Ia), (II) and/or (IIa) is a hole-transporting or hole-injecting material.
26. Optoelectronic device in accordance with item 24 or 25, wherein the compound of formula (I) or (Ia) and/or (II) or (IIa) is contained in an organic film in the optoelectronic device.
27. Optoelectronic device in accordance with any of items 24 to 26, wherein the optoelectronic device is selected from an organic light emitting diode (OLED) and a photovoltaic device.
28. Optoelectronic device in accordance with item 27, wherein the optoelectronic device is a solar cell.
29. Optoelectronic device in accordance with item 28, wherein the solar cell is a perovskite solar cell.

### Examples

### Synthesis of EDOT-Amide-TPA

EDOT-Amide-TPA as a hole-transporting or hole-injecting material in accordance with the invention was prepared by condensing 4-amino-4',4",-dimethoxytriphenylamine with 2,3-dihydrothieno[3,4-b][1,4]dioxine-5,7-dihydrochloric acid in the presence of triethylamine in line with the following reaction scheme. The product was obtained in an excellent yield of 86% and in high purity.

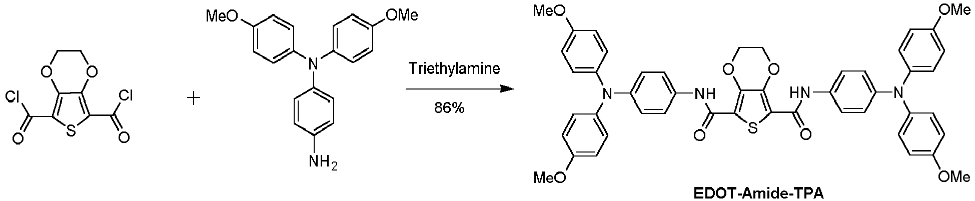

4-Amino-4',4",-dimethoxytriphenylamine and 3-dihydrothieno[3,4-*b*][1,4]dioxine-5,7-dihydro-chloric acid were synthesized according to literature (Petrus, M. L.; Bein, T.; Dingemans, T. J.; Docampo, P. J. Mater. Chem. A 2015, 3 (23), 12159; Wilsens, C. H. R. M.; Deshmukh, Y. S.; Noordover, B. A. J.; Rastogi, S. Macromolecules 2014, 47 (18), 6196).

2,3-Dihydrothieno[3,4-b][1,4]dioxine-5,7-dihydrochloric acid (1.36 mmol) was dissolved in THF (20 mL) and 4-amino-4',4",-dimethoxytriphenylamine (1 g, 3.1 mmol) and triethylamine (0.1 mL) were added. The solution was refluxed for 1.5 hours and stirred overnight at room temperature. The orange product was collected by filtration, washed and dried under vacuum (0.99 g, 1.2 mmol, 86%).

### Synthesis of Ph-Amide-OMeTPA

Ph-Amide-TPA was prepared by condensing 4-amino-4',4",-dimethoxytriphenylamine with terephthaloyl dichloride in the presence of triethylamine in line with the following reaction scheme.

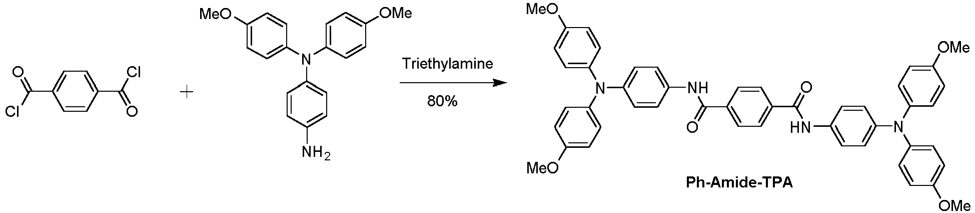

### a) Terephthaloyl dichloride

Thionyl chloride (0.95 mL, 13.2 mmol, 2.3 eq) was added dropwise to a solution of terephtalic acid (0.95 g, 5.7 mmol, 1.0 eq) and DMF (a few drops) in dry THF (80 mL). The reaction mixture was stirred at 80 °C for 2 h. Afterwards, the solution was cooled down to room temperature and the remaining SOCl₂ and THF were removed in vacuo to give the product as a yellowish powder, which was used without further purification for the synthesis of Ph-Amide-OMeTPA.

b) The before synthesized acid chloride (0.30 g, 1.5 mmol, 1.0 eq) and 4-amino-4',4",-dimethoxytriphenylamine (1.10 g, 3.5 mmol, 2.3 eq.) were dissolved in dry THF and triethyl amine (TEA, a few drops) was added to the solution. The reaction mixture was heated to reflux for 1.5 h and afterwards cooled down and stirred at room temperature over night to give a yellowish suspension. The precipitate was then filtered off, washed with isopropanol, water and hexane and dried to give Ph-Amide-OMeTPA as a yellow powder (0.92 g, 1.2 mmol, 80%).

### Synthesis of Py-Amide-OMeTPA

Py-Amide-OMeTPA was prepared by condensing 4-amino-4',4",-dimethoxytriphenylamine with 2,6-pyridinedicarbonyl dichloride in the presence of triethylamine in line with the following reaction scheme.

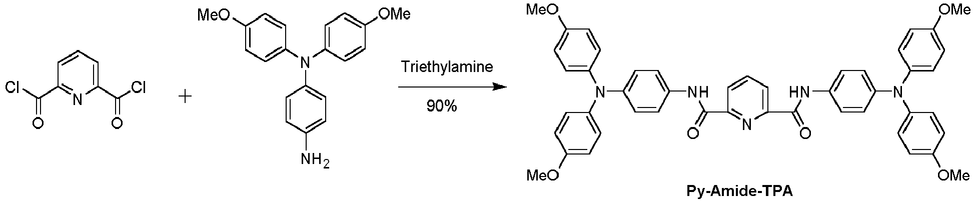

2,6-Pyridinedicarbonyl dichloride (0.34 g, 1.7 mmol, 1.0 eq) and 4-amino-4',4",-dimethoxytriphenylamine (1.11 g, 3.5 mmol, 2.1 eq.) were dissolved in dry THF and triethylamine (a few drops) was added to the solution. The reaction mixture was heated to reflux for 1.5 h and afterwards cooled down and stirred at room temperature over night to give a yellowish suspension. The precipitate was then filtered off, washed with isopropanol, water and hexane and dried to give Py-Amide-OMeTPA as a yellow powder (1.16 g,1.5 mmol, 90%).

### Synthesis of diAmide-OMeTPA

diAmide-OMeTPA was prepared by condensing 4-amino-4',4",-dimethoxytriphenylamine with oxalyl chloride in the presence of triethylamine in line with the following reaction scheme.

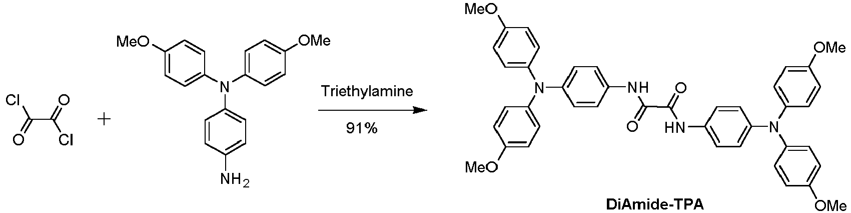

Oxalyl chloride (0.14 mL, 1.6 mmol, 1.0 eq) and 4-Amino-4',4",-dimethoxytriphenylamine (1.23 g, 3.8 mmol, 2.4 eq.) were dissolved in dry THF and triethylamine (a few drops) was added to the solution. The reaction mixture was heated to reflux for 1.5 h and afterwards cooled down and stirred at room temperature overnight to give a yellowish suspension. The precipitate was then filtered off, washed with isopropanol, water and hexane and dried to give diAmide-OMeTPA as a yellow powder (1.01 g,1.5 mmol, 91%).

### Preparation of Optoelectronic Device

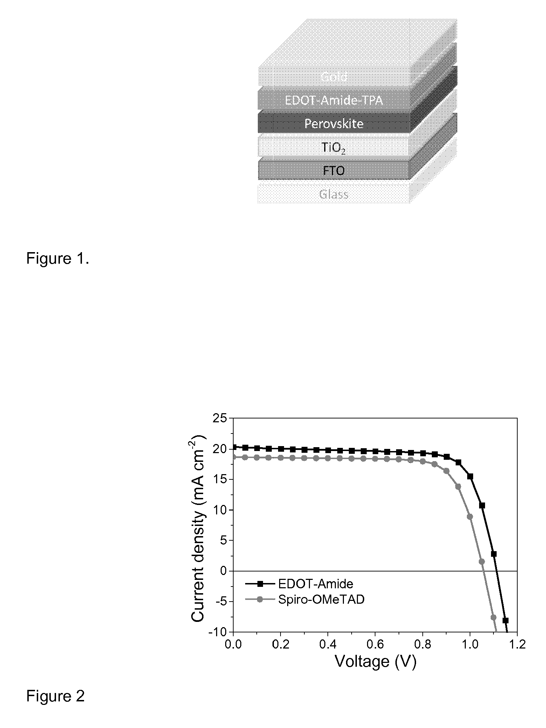

We prepared planar methylammonium lead iodide based perovskite solar cells with a setup schematically illustrated in Fig. 1 employing EDOT-Amide-TPA as hole transporting material in accordance with the invention and compared the performance to a device comprising the state-of-the-art Spiro-OMeTAD.

### Synthesis of Methylammonium iodide (MAI)

An HI solution (10 mL, 76 mmol) was dropwise added to a methylamine solution (24 mL, 193 mmol) diluted in absolute ethanol (100 mL) at 0 °C under stirring. After further stirring for 2 h at room temperature, the solvent was removed by using a rotary evaporator and the colorless precipitate was recrystallized from absolute ethanol. Finally, the colorless MAI crystals were filtrated, washed with diethyl ether and dried in vacuum overnight.

### Device fabrication

For the planar device fabrication, FTO (fluorine-doped tin oxide) coated glass slides (Pilkington, 7 Ω/sq) were used. In order to prevent shunting upon contacting the final device, the substrates were patterned by etching with zinc powder and 3 M HCl solution. Then the etched FTO-glass substrates were cleaned successively with deionized water, a 2% Hellmanex detergent solution, acetone, ethanol and finally treated with oxygen plasma for 5 min. An electron transporting TiO₂ compact layer with 20 nm thickness was deposited on the substrate via a sol-gel approach. Therefore a mixture of 2 M HCl (35 µL) and anhydrous IPA (2.53 mL) was added dropwise to a solution of titanium isopropoxide (370 µL) in IPA (2.53 mL) under vigorous stirring. The clear TiOₓ sol-gel solution was spin-coated dynamically on the FTO substrates at 2000 rpm for 45 s, followed by annealing at 150 °C for 10 min on a hotplate. Finally, the TiO₂ compact layer was completed by sintering at 500 °C in air for 45 min and the substrates were cut into pieces of 3 × 3 cm².

Thin films of MAPI perovskite were prepared in a glovebox under dry nitrogen atmosphere, by following a fast deposition-crystallization protocol. MAI (0.4 g, 2.5 mmol) and Pbl₂ (1.21 g, 2.6 mmol) were dissolved in 2 mL DMF/DMSO (4:1) under stirring at 100 °C. The yellow solution was allowed to cool down to room temperature and filtrated through a 0.45 µm syringe filter, affording the precursor solution of MAPI. Then 80 µL MAPI solution was dynamically spin-coated on the substrate at 1000 rpm for 10 s. followed by a second step at 5000 rpm for 30 s. In a second step, 15 s after the deposition of the MAPI precursor solution, 400 µL chlorobenzene was introduced to the spinning film in order to promote MAPI crystallization. The sample was annealed on a hotplate at 40 °C for 40 min followed by annealing at 100 °C for 10 min to remove residual solvents and homogeneous, dark-brown MAPI films with a thickness of -300 nm were obtained.

For the hole transporter layer, a solution of 2,2',7,7'-tetrakis-(N,N-di-p-methoxyphenyl-amine)-9,9'-spirobifluorene (Spiro-OMeTAD, comparative compound) in anhydrous chlorobenzene (75 mg/ml at R.T.) and EDOT-Amide-TPA in anhydrous chlorobenzene (10 mg/ml dissolved at 100 °C) were prepared. To these solutions 4-*tert*-butylpyridine (10 µL) and a 170 mg/ml lithium bistrifluoromethanesulfonimidate (Li-TFSI, 30 µL) solution in acetonitrile were added to 1 mL of the solutions. Hole-transporters were deposited on the device substrate by spin-coating at 1500 rpm for 40 s, the Spiro-OMeTAD solution was at R.T. while the EDOT-Amide-TPA solution was at 100 °C. The samples were stored overnight in a desiccator under air atmosphere with approximately 30% relative humidity. Finally, a 40 nm Au layer was deposited through a patterned shadow mask by thermal evaporation at 10⁻⁷ mbar and a deposition rate of 0.1 nm/s in order to form the back electrode.

### Characterization

Current-Voltage (*J-V*) characteristics of the perovskite solar cells were measured under standard solar simulator conditions using a Newport OrielSol 2A solar simulator with a Keithley 2401 source meter. The devices were illuminated through a shadow mask, yielding an active area of 0.0813 cm². The *J-V* curves were recorded under standard AM 1.5G illumination from a xenon lamp, calibrated to a light intensity of 100 mW cm⁻² with a Fraunhofer ISE certified silicon diode. The input bias voltage was scanned from -1.5 V to 0 V in 0.05 V steps with a rate of 0.5 V s⁻¹.

Hole-only devices were prepared by thermal deposition of MoOₓ (10 nm) under vacuum (~1· 10⁻⁶ mbar) on a pre-cleaned ITO patterned glass substrates. The samples were exposed to air and stored in a nitrogen-filled glovebox. Hole-transporters were deposited on the device substrate by spin-coating at 1500 rpm for 40 s, the Spiro-OMeTAD solution (75 mg/mL in chlorobenzene) was at R.T. while the EDOT-Amide-TPA solution (10 mg/mL in chlorobenzene) was at 100 °C. A top electrode of MoOx (10 nm) followed by a layer of gold (100 nm) were deposited under vacuum (~1· 10-6 mbar). The active area is 16 mm². Current-voltage characteristics were recorded in air using a Metrohm potentiostat (PGSTAT302N).

In order to determine the conductivity the hole transporting layer was spincoated on a pre-cleaned glass substrate as described above, and different amounts of additives (LiTFSI) were added to the solution. On top, planar interdigitating electrodes (gold) were thermally depositited under vacuum (~1· 10-6 mbar). The J-V curves were recorded using a Keithley 2401 source meter.

Devices comprising EDOT-Amide-TPA show enhanced short-circuit (*J*_{sc}) current and open-circuit voltage (*V*_{oc}), which lead to an increase in the device efficiency from 15.1% for Spiro-OMeTAD to over 17% for the EDOT-Amide-TPA material. The *JV*-curves are shown in Figure 2, while the photovoltaic parameters (*J*_{sc}, *V*_{oc}, fill factor (FF) and power conversion efficiency (PCE)) are summarized in Table 1 below.

**Table 1: Device performance of planar heterojunction perovskite solar cells comprising EDOT-Amide-TPA and Spiro-OMeTAD as hole transporting materials.**

| | ***J*_{sc} (mA/cm²)** | ***V*_{oc} (V)** | **FF (%)** | **PCE (%)** |
|---|---|---|---|---|
| Spiro-OMeTAD | 18.7 | 1.06 | 76 | 15.1 |
| EDOT-Amide-TPA | 20.4 | 1.11 | 75 | 17.2 |

The hole-transporting materials in accordance with the invention were employed in planar NH₃CH₃Pbl₃ perovskite photovoltaics, where efficiencies exceeding 17%, outperforming the current state-of-the-art material Spiro-OMeTAD (16%) on a like-to-like comparison, could be obtained. The materials can be easily prepared in a cost effective condensation reaction which results in over ten times lower costs than the state-of-the-art. In addition, the high synthetic accessibility of these small-molecules also reduces the (toxic) chemical waste and therefore greatly reduces its environmental impact. For example, no halogenated solvents are required and water or HCl may be the only side product in this reaction.. All these elements make these novel materials excellent candidates for industrial applications not just in perovskite solar cells but also for other optoelectronic applications.

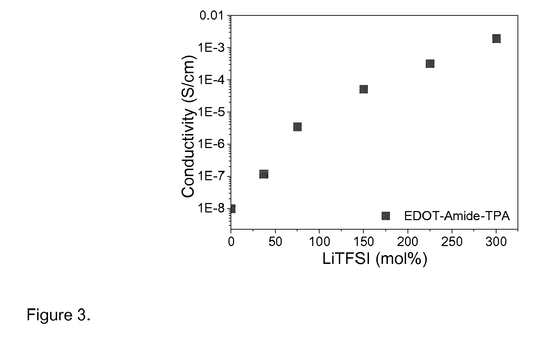

The hole-transporting layer using EDOT-Amide-TPA in the above perovskite solar cell has a hole mobility of 3.9·10⁻⁵ cm²/Vs which is comparable to the state-of-the-art material Spiro-OMeTAD (4.0·10⁻⁵ cm²/Vs), which was determined in a hole-only device. The conductivity of pristine EDOT-Amide-TPA (9.9·10⁻⁹ S/cm) is slightly lower than that of Spiro-OMeTAD (8.7·10⁻⁸ S/cm), however, after doping EDOT-Amide-TPA with LiTFSI we obtained conductivities exceeding 10⁻³ S/cm as shown in Fig. 3.

### Figures

Figure 1 is a schematic illustration of the device architecture of a planar heterojunction perovskite solar cell comprising EDOT-Amide-TPA as hole-transporting material.
Figure 2 shows the J-V characteristics under AM1.5G solar illumination (100 W/cm²) of planar heterojunction perovskite solar cells comprising EDOT-Amide-TPA (hole-transporting material in accordance with the invention) and Spiro-OMeTAD (reference material) as hole-transporting materials.
Figure 3 shows the conductivity of EDOT-Amide-TPA alone and in combination with LiTFSI as a conductivity improving additive.

## Claims

1. Use of a compound of the formula (I) and/or a compound of the formula (II) 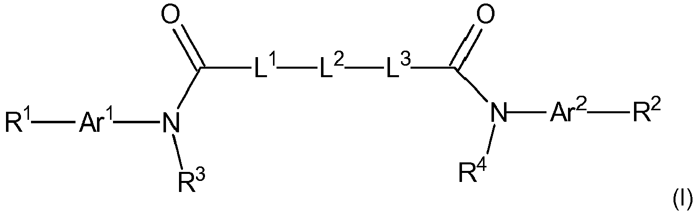 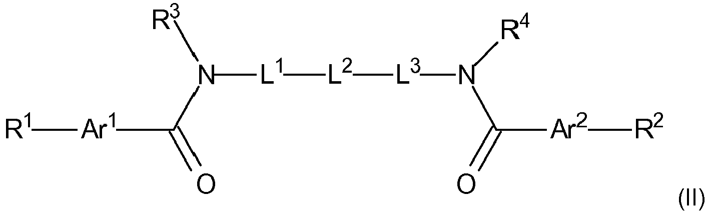 as a hole-transporting or hole-injecting material in an optoelectronic device,
wherein:
Ar¹ and Ar² independently represent an optionally substituted arylene group, an optionally substituted heteroarylene group or an optionally substituted fluorenylene group;
R¹ and R² independently represent hydrogen or a group -NAr⁶Ar⁷, wherein Ar⁶ and Ar⁷ are independently an optionally substituted aryl group, an optionally substituted heteroaryl group, or an optionally substituted fluorenyl group, and wherein a direct covalent bond may be present between Ar⁶ and Ar⁷ such that a fused heterocyclic ring system is formed which contains Ar⁶, Ar⁷ and the nitrogen atom to which they are attached;
L¹ and L³ independently represent a bond, an optionally substituted arylene group, an optionally substituted fluorenylene group, or an optionally substituted heteroarylene group;
L² represents a bond, an optionally substituted arylene group, an optionally substituted heteroarylene group, an optionally substituted fluorenylene group, or a group -Ar³-N(Ar⁴)-Ar⁵-, wherein Ar³ and Ar⁵ independently represent an optionally substituted arylene group, and Ar⁴ represents an optionally substituted aryl group;
R³ and R⁴ independently represent hydrogen, an alkyl group or an aryl group;
and wherein any optionally substituted aryl group, arylene group, heteroaryl group, heteroarylene group, fluorenyl group and fluorenylene group may be substituted by one or more substituents, independently selected from -CN, alkyl, alkoxy, aryl, heteroaryl, halogen, and amino, and wherein two adjacent optional substituents selected from alkyl, alkoxy and amino may be taken together to form a ring fused with the aryl group, arylene group, heteroaryl group, heteroarylene group, fluorenyl group and fluorenylene group to which they are attached.

2. Use in accordance with claim 1, wherein
L¹ and L³ represent a bond;
L² represents an optionally substituted heteroarylene group.

3. Use in accordance with claim 1, wherein the compound of formula (I) is a compound of formula (Ia) and the compound of formula (II) is a compound of formula (IIa): 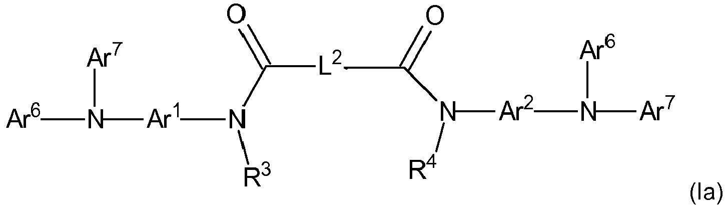 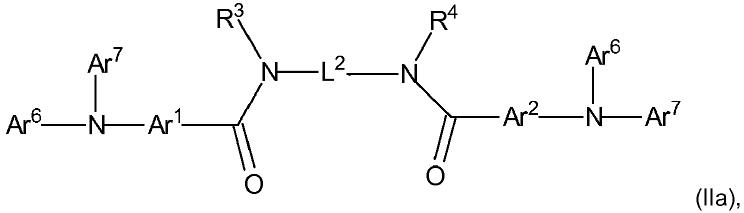 wherein:
L² is defined as in claim 2, and R³, R⁴, Ar¹, Ar², Ar⁶ and Ar⁷ are defined as in claim 1.

4. Use in accordance with any of claims 1 to 3, wherein Ar¹ and Ar² independently represent an optionally substituted arylene group.

5. Use in accordance with any of claims 1 to 4, wherein R¹ and R² independently represent a group -NAr⁶Ar⁷, wherein Ar⁶ and Ar⁷ are independently an optionally substituted aryl group, and wherein a direct covalent bond may be present between Ar⁶ and Ar⁷ such that a fused heterocyclic ring system is formed which contains Ar⁶, Ar⁷ and the nitrogen atom to which they are attached.

6. Use in accordance with any of claims 1 to 5, wherein R³ and R⁴ are hydrogen.

7. Use in accordance with any of claims 1 to 6, wherein L² represents a moiety of the formula (III): 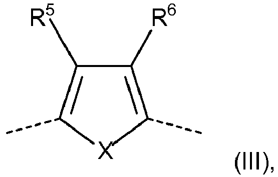 wherein
X is a heteroatom selected from O, Se and S, and
R⁵ and R⁶ are independently selected from H, -CN, alkyl, alkoxy, aryl, heteroaryl, halogen and amino, and two substituents selected from alkyl, alkoxy and amino may be taken together to form a ring structure fused with the heteroarylene group to which they are attached, and the dashed lines indicate the position where covalent bonds are formed by the divalent moiety L² to its neighboring atoms.

8. Use in accordance with any of claims 1 to 7, wherein L² represents a moiety of the formula (IV): 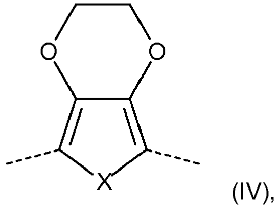 wherein
X is a heteroatom selected from O, Se and S, and the dashed lines indicate the position where covalent bonds are formed by the divalent moiety L² to its neighboring atoms.

9. Use in accordance with claim 12 or 13, wherein X is S.

10. Use in accordance with any of claims 1 to 9, wherein the optoelectronic device is selected from an organic light emitting diode (OLED) and a photovoltaic device.

11. An optoelectronic device, comprising one or more compounds of formula (I), (Ia), (II) and/or (IIa) as defined in any of claims 1 to 9.

12. Optoelectronic device in accordance with claim 11, wherein the optoelectronic device is selected from an organic light emitting diode (OLED) and a photovoltaic device.

13. Optoelectronic device in accordance with claim 12, wherein the optoelectronic device is a solar cell.

14. Optoelectronic device in accordance with claim 13, wherein the solar cell is a perovskite solar cell.
